# EUROPEAN PATENT SPECIFICATION

(11) **EP 2 205 292 B1**
(45) Date of publication and mention of the grant of the patent: **15.05.2013**
(21) Application number: 08832630.1
(22) Date of filing: 19.09.2008
(51) Int. Cl.: A61L 31/10, A61L 31/14, A61L 31/16

(54) **THERAPEUTIC AGENT-ELUTING MEDICAL DEVICES HAVING TEXTURED POLYMERIC SURFACES**
EIN THERAPEUTISCHES MITTEL FREISETZENDE MEDIZINPRODUKTE MIT STRUKTURIERTEN POLYMER-OBERFLÄCHEN
DISPOSITIFS MÉDICAUX ÉLUANT UN AGENT THÉRAPEUTIQUE À SURFACES POLYMÉRIQUES TEXTURÉES

(30) Priority: 21.09.2007 US 994724 P
(43) Date of publication of application: 14.07.2010
(73) Proprietor: Boston Scientific Scimed, Inc., Maple Grove, MN 55311-1566 (US)
(72) Inventor: FLANAGAN, Alden, Galway (IE); BODEN, Mark, Harrisville RI 02830 (US); STRICKLER, Frederick, H., Natick MA 01760 (US); TENNEY, Barron, W., Haverhill MA 01832 (US)
(74) Representative: Hauck Patent- und Rechtsanwälte
(86) International application number: PCT/US2008/077101
(87) International publication number: WO 2009/039429

(56) References cited:
- EP-A2- 0 749 730
- WO-A1-03/026718
- US-A1- 2002 173 838
- US-A1- 2004 093 068
- US-A1- 2004 098 119

## Description

### FIELD OF THE INVENTION

The present invention relates to medical devices and more particularly to therapeutic-agent-eluting medical devices having textured polymeric surfaces.

### BACKGROUND OF THE INVENTION

The *in vivo* delivery of biologically active agents within the body of a patient is common in the practice of modern medicine. *In vivo* delivery of biologically active agents is often implemented using medical devices that may be temporarily or permanently placed at a target site within the body. These medical devices can be maintained, as required, at their target sites for short or prolonged periods of time, delivering biologically active agents at the target site.

Many state of the art medical devices have biostable or biodegradable polymeric coatings, which serve as reservoirs for the release of one or more one or more therapeutic agents. For example, therapeutic-agent-eluting coronary stents, such as those commercially available from Boston Scientific Corp. (TAXUS®), Johnson & Johnson (CYPHER®), and others are frequently prescribed for use in maintaining blood vessel patency. These products are based on metallic balloon expandable stents with biostable polymer coatings, which release antiproliferative therapeutic agents at a controlled rate and total dose, for preventing restenosis of the blood vessel.

Various types of polymeric materials have been used as reservoir matrices into which therapeutic agents are placed. It has been found that multiphase polymer systems including polymer blends and block copolymers are particularly advantageous as medical device coatings. For example, CYPHER stents are coated with a thin layer of a blend of poly(w-butyl methacrylate) and ethylene-vinyl acetate copolymer and contain sirolimus as an anti-restenotic agent. R. Virmani et al., Circulation 2004 Feb 17, 109(6) 701-5. The polymer coating technology in the TAXUS therapeutic-agent-eluting stent contains a thermoplastic elastomer poly(styrene-[epsilon]-isobutylene-[epsilon]-styrene) (SIBS) and paclitaxel. S. Ranadc et al., "Physical characterization of controlled release of paclitaxel from the TAXUS(TM) Express2(TM) drug-eluting stent," Journal of Biomedical Materials Research Part A 7 IA (2004) 625-634.

Such devices can be made using a variety of methods. For example, in a current process for forming TAXUS products, the outer surface of a stainless steel coronary stent is sprayed with a solution of that contains solvent, paclitaxel and STBS. Although the solution is spayed on the outside of the stent, the stent is ultimately encapsulated with the polymeric coating due to through-strut spraying and wicking of the solution around the stent struts. The result of such a process is schematically illustrated, for example, in Figs. IA and IB. Fig. IA is a schematic perspective view of a stent 100 which contains a number of interconnected struts 100s. Fig. IB is a cross-section taken along line b- b of strut 110s of stent 100 of Fig. IA, and shows a stainless steel stent substrate 110 and a paclitaxel-containing polymeric coating 120, which covers the abluminal surface 110a, luminal surface 1101 and side surfaces 100s of the substrate 110, encapsulating the same.

Document US 2002/173838 A1 teaches a stent in which a perivascular drug delivery matrix is applied. The matrix may consist of a non-biodegradable polymer, a biodegradable polymer, or an absorbable biopolymer. The endocoronary stent has a textured surface, which may be polymeric or a powdered metal.

Document EP 0 749 730 A2 teaches a stent which comprises a sheet of material curled into a cylinder and having a surface texture made of a plurality of protrusions and an interior surface containing a plurality of apertures.

Document WO 03/026718 A1 teaches a stent having a layer of a bioactive material posited directly upon the roughened or textured surface of the base material of the structure. The surface is roughened or textured by etching or by abrasion with sodium bicarbonate or another suitable grit.

### SUMMARY OF THE INVENTION

In accordance with an aspect of the present invention, medical devices are provided which comprise the following: (a) a substrate having first and second surfaces, (b) a therapeutic-agent-eluting polymeric layer disposed over the first substrate surface and (c) a textured polymeric layer that is textured to promote cell attachment, cell growth, or both disposed over the second surface.

The medical device is a stent, wherein the first surface may be the abluminal surface, and the second surface may be the luminal surface.

In this regard, an advantage of the present invention is that a therapeutic-agent- eluting stent may be provided, which allows endothelium regeneration (healing) at a rate greater than current coated stents. For example, it is advantageous to provide the outer abluminal surface of a stent with an antiproliferative-agent-releasing polymeric coating to combat smooth muscle cell proliferation, which may, for example, lead to impeded blood flow and interfere with the formation of a healthy endothelium if not controlled. Positioning antiproliferative therapeutic agents primarily or exclusively on the abluminal surface is advantageous as it may lessen the amount of antiproliferative therapeutic agent that is required to combat smooth muscle proliferation and/or it may minimize or eliminate the release of antiproliferative therapeutic agents from the luminal surface, which agents might otherwise interfere with endothelium development and be transported to other parts of the body via the bloodstream. Moreover, the present invention is advantageous in that providing a textured luminal surface may promote healthy endothelial cell growth on the luminal surface.

The above and other aspects, embodiments and advantages of the present invention will become immediately apparent to those of ordinary skill in the art upon review of the Detailed Description and Claims to follow.

### BRIEF DESCRIPTION OF THE DRAWINGS

Fig. 1A is a schematic perspective view of a stent in accordance with the prior art. Fig. 1B is a schematic cross-sectional view taken along line b--b of Fig. 1A

Fig. 2 is a high resolution atomic force microscopy topography image of a polymer brush in accordance with the prior art.

Fig 3 is a schematic cross sectional view of a stent strut in accordance with an embodiment of the present invention.

Fig 4 is a schematic cross sectional view of a stent strut in accordance with another embodiment of the present invention.

Fig. 5 is a schematic illustration of a luminal surface of a portion of a stent strut, in accordance with an embodiment of the invention.

### DETAILED DESCRIPTION OF THE INVENTION

According to an aspect of the present invention, medical devices are provided which comprise the following: (a) a substrate having first and second surfaces, (b) a therapeutic-agent-eluting polymeric layer disposed over the first substrate surface and (c) a textured polymeric layer that is textured to promote cell attachment, cell growth, or both disposed over the second surface.

The medical device is a stent. In particular embodiments, the first and second surfaces are abluminal and luminal surfaces, respectively, the first therapeutic agent is an agent that inhibits the proliferation of smooth muscle cells, and the textured polymeric layer is textured to promote attachment and/or proliferation of endothelial cells. The textured polymeric layer may optionally comprise a further therapeutic agent, for example, an agent that promotes the formation of endothelial cells or precursor endothelial cells, among others.

This particular embodiment thus provides a stent that has a textured luminal surface (with or without a therapeutic agent), and a therapeutic-agent-eluting abluminal surface. The textured luminal surface is provided to promote the attachment and/or proliferation of endothelial cells (e.g., which may grow over the stent struts to form a confluent layer of endothelial cells), while the abluminal surface may release smooth- muscle-cell-inhibiting therapeutic agent into the surrounding vascular tissue where it is desired. Consequently, the smooth-muscle-cell-inhibiting therapeutic agent will not be released in significant quantities from the luminal surface, where it may interfere with endothelial cell growth or where it may be released into the bloodstream, for example, causing undesirable systemic effects.

"Therapeutic agents," "biologically active agents," "drugs," "pharmaceutically active agents," "pharmaceutically active materials," and other related terms may be used interchangeably herein and include genetic therapeutic agents, non-genetic therapeutic agents and cells. A wide variety of therapeutic agents can be employed in conjunction with the present invention including those used for the treatment of a wide variety of diseases and conditions (i.e., the prevention of a disease or condition, the reduction or elimination of symptoms associated with a disease or condition, or the substantial or complete elimination of a disease or condition). Numerous therapeutic agents are listed below.

As used herein a "layer" of a given material is a region of that material whose thickness is small compared to both its length and width. As used herein a layer need not be planar, for example, taking on the contours of an underlying substrate. As used herein a layer can be discontinuous, providing only partial coverage of the underlying substrate. Terms such as "film," "layer" and "coating" may be used interchangeably herein.

As used herein, "polymers" are molecules containing multiple copies (e.g., from 3 to 5 to 10 to 25 to 50 to 100 to 250 to 500 to 1000 or more copies) of one or more constitutional units, commonly referred to as monomers. As used herein, the term "monomers" may refer to the free monomers or monomers that are incorporated into polymers, with the distinction being clear from the context in which the term is used. Polymers may take on a number of configurations, which may be selected, for example, from cyclic, linear and branched configurations, among others. Branched configurations include star-shaped configurations (e.g., configurations in which three or more chains emanate from a single branch point), comb configurations (e.g., configurations having a main chain and a plurality of side chains, also referred to as "graft" or "bottlebrush" configurations), dendritic configurations (e.g., arborescent and hyperbranched polymers), and so forth.

As used herein, "homopolymers" are polymers that contain multiple copies of a single constitutional unit. "Copolymers" are polymers that contain multiple copies of at least two dissimilar constitutional units, examples of which include random, statistical, gradient, tapered, periodic (e.g., alternating) and block copolymers. As used herein, "block copolymers" are copolymers that contain two or more polymer blocks that differ in composition, for instance, because a constitutional unit (i.e., a monomer) is found in one polymer block that is not found in another polymer block. As used herein, a "polymer block" or "block" is a grouping of constitutional units (e.g., 3 to 5 to 10 to 25 to 50 to 100 to 250 to 500 to 1000 or more units) and may correspond to a portion of a polymer (e.g., as in a block copolymer) or an entire polymer. Blocks can be unbranched or branched. Blocks can contain a single type of constitutional unit (also referred to herein as "homopolymeric blocks") or multiple types of constitutional units (also referred to herein as "copolymeric blocks") which may be present, for example, in a random, statistical, gradient, or periodic (e.g., alternating) distribution.

As used herein, a "chain" is a linear polymer or a portion thereof, for example, a linear block.

Examples of medical devices benefiting from the present invention vary widely and include implantable or insertable medical devices, for example, stents (including coronary vascular stents, peripheral vascular stents, cerebral, urethral, ureteral, biliary, tracheal, gastrointestinal and esophageal stents), stent coverings, stent grafts, vascular grafts, abdominal aortic aneurysm (AAA) devices (e.g., AAA stents, AAA grafts), vascular access ports, dialysis ports, catheters (*e.g*., urological catheters or vascular catheters such as balloon catheters and various central venous catheters), guide wires, balloons, filters (*e.g*., vena cava filters and mesh filters for distil protection devices), embolization devices including cerebral aneurysm filler coils (including Guglilmi detachable coils and metal coils), cerebral aneurysm repair devices, septal defect closure devices, myocardial plugs, patches, pacemakers, lead coatings including coatings for pacemaker leads, defibrillation leads, and coils, ventricular assist devices including left ventricular assist hearts and pumps, total artificial hearts, shunts, valves including heart valves and vascular valves, anastomosis clips and rings, cochlear implants, tissue bulking devices, and tissue engineering scaffolds for cartilage, bone, skin and other in vivo tissue regeneration, gastric balloons, ocular devices, sutures, suture anchors, tissue staples and ligating clips at surgical sites, cannulae, metal wire ligatures, urethral slings, hernia "meshes", artificial ligaments, orthopedic prosthesis such as bone grafts, bone plates, fins and fusion devices, spinal disc prosthesis, joint prostheses, orthopedic fixation devices such as interference screws in the ankle, knee, and hand areas, tacks for ligament attachment and meniscal repair, rods and pins for fracture fixation, screws and plates for craniomaxillofacial repair, dental implants, or other devices that are implanted or inserted into the body and from which therapeutic agent is released.

The medical devices of the present invention include, for example, implantable and insertable medical devices that are used for systemic treatment, as well as those that are used for the localized treatment of any tissue or organ. Non-limiting examples are tumors; organs including the heart, coronary and peripheral vascular system (referred to overall as "the vasculature"), the urogenital system, including kidneys, bladder, urethra, ureters, prostate, vagina, uterus and ovaries, eyes, ears, spine, nervous system, lungs, trachea, esophagus, intestines, stomach, brain, liver and pancreas, skeletal muscle, smooth muscle, breast, dermal tissue, cartilage, tooth and bone. Examples further include any medical device that is exposed to flowing blood.

As used herein, "treatment" refers to the prevention of a disease or condition, the reduction or elimination of symptoms associated with a disease or condition, or the substantial or complete elimination of a disease or condition. Subjects are vertebrate subjects, more typically mammalian subjects, including human subjects, pets and livestock.

Substrate materials for the medical devices of the present invention may vary widely in composition and are not limited to any particular material. They can be selected from a range of biostable materials and biodisintegrable materials (i.e., materials that, upon placement in the body, are dissolved, degraded, resorbed, and/or otherwise removed from the placement site), including (a) organic materials (i.e., materials containing organic species, typically 50 wt% or more, for example, from 50 wt% to 75 wt% to 90 wt% to 95 wt% to 97.5 wt% to 99 wt% or more) such as polymeric materials (i.e., materials containing polymers, typically 50 wt% or more polymers, for example, from 50 wt% to 75 wt% to 90 wt% to 95 wt% to 97.5 wt% to 99 wt% or more) and biologics, (b) inorganic materials (i.e., materials containing inorganic species, typically 50 wt% or more, for example, from 50 wt% to 75 wt% to 90 wt% to 95 wt% to 97.5 wt% to 99 wt% or more), such as metallic materials (i.e., materials containing metals, typically 50 wt% or more, for example, from 50 wt% to 75 wt% to 90 wt% to 95 wt% to 97.5 wt% to 99 wt% or more) and non-metallic inorganic materials (i.e., materials containing non-metallic inorganic materials, typically 50 wt% or more, for example, from 50 wt% to 75 wt% to 90 wt% to 95 wt% to 97.5 wt% to 99 wt% or more) (e.g., including carbon, semiconductors, glasses and ceramics, which may contain various metal- and non-metal-oxides, various metal- and non-metal-nitrides, various metal- and non-metal-carbides, various metal- and non-metal-borides, various metal- and non-metal-phosphates, and various metal- and non-metal-sulfides, among others), and (c) hybrid materials (e.g., hybrid organic/inorganic materials, for instance, polymer/metallic-inorganic hybrids and polymer/non-metallic-inorganic hybrids).

Specific examples of inorganic non-metallic materials may be selected, for example, from materials containing one or more of the following: metal oxide ceramics, including aluminum oxides and transition metal oxides (e.g., oxides of titanium, zirconium, hafnium, tantalum, molybdenum, tungsten, rhenium, iron, niobium, and iridium); silicon; silicon-based ceramics, such as those containing silicon nitrides, silicon carbides and silicon oxides (sometimes referred to as glass ceramics); calcium phosphate ceramics (e.g., hydroxyapatite); carbon; and carbon-based, ceramic-like materials such as carbon nitrides.

Specific examples of metallic materials may be selected, for example, from metals such as gold, iron, niobium, platinum, palladium, iridium, osmium, rhodium, titanium, tantalum, tungsten, ruthenium, and magnesium, among others, and metal alloys such as those comprising iron and chromium (e.g., stainless steels, including platinum-enriched radiopaque stainless steel), niobium alloys, titanium alloys, alloys comprising nickel and titanium (e.g., Nitinol), alloys comprising cobalt and chromium, including alloys that comprise cobalt, chromium and iron (e.g., elgiloy alloys), alloys comprising nickel, cobalt and chromium (e.g., MP 35N), alloys comprising cobalt, chromium, tungsten and nickel (e.g., L605), alloys comprising nickel and chromium (e.g., inconel alloys), and biodisintegrable alloys including alloys of magnesium, zinc and/or iron (and their alloys with combinations of Ce, Ca, Zn, Zr and Li), among others.

Specific examples of organic materials include polymers (biostable or biodisintegrable) and other high molecular weight organic materials, and may be selected, for example, from suitable materials containing one or more of the following: polycarboxylic acid polymers and copolymers including polyacrylic acids; acetal polymers and copolymers; acrylate and methacrylate polymers and copolymers (e.g., n-butyl methacrylate); cellulosic polymers and copolymers, including cellulose acetates, cellulose nitrates, cellulose propionates, cellulose acetate butyrates, cellophanes, rayons, rayon triacetates, and cellulose ethers such as carboxymethyl celluloses and hydroxyalkyl celluloses; polyoxymethylene polymers and copolymers; polyimide polymers and copolymers such as polyether block imides, polyamidimides, polyesterimides, and polyetherimides; polysulfone polymers and copolymers including polyarylsulfones and polyethersulfones, polyamide polymers and copolymers including nylon 6,6, nylon 12, polyether-block co-polyamide polymers (e.g., Pebax® resins), polycaprolactams and polyacrylamides; resins including alkyd resins, phenolic resins, urea resins, melamine resins, epoxy resins, allyl resins and epoxide resins; polycarbonates; polyacrylonitriles; polyvinylpyrrolidones (cross-linked and otherwise); polymers and copolymers of vinyl monomers including polyvinyl alcohols, polyvinyl halides such as polyvinyl chlorides, ethylene-vinylacetate copolymers (EVA), polyvinylidene chlorides, polyvinyl ethers such as polyvinyl methyl ethers, vinyl aromatic polymers and copolymers such as polystyrenes, styrene-maleic anhydride copolymers, vinyl aromatic-hydrocarbon copolymers including styrene-butadiene copolymers, styrene-ethylene-butylene copolymers (e.g., a polystyrene-polyethylene/butylene-polystyrene (SEBS) copolymer, available as Kraton® G series polymers), styrene-isoprene copolymers (e.g., polystyrene-polyisoprene-polystyrene), acrylonitrile-styrene copolymers, acrylonitrile-butadienestyrene copolymers, styrene-butadiene copolymers, styrene-isobutylene copolymers (e.g., polyisobutylene-polystyrene block copolymers such as SIBS), and isobutylene-methacrylate copolymers including polyisobutylene-polymethacrylate block copolymers, polyvinyl ketones, polyvinylcarbazoles, and polyvinyl esters such as polyvinyl acetates; polybenzimidazoles; ionomers; polyalkyl oxide polymers and copolymers including polyethylene oxides (PEO); polyesters including polyethylene terephthalates, polybutylene terephthalates and aliphatic polyesters such as polymers and copolymers of lactide (which includes lactic acid as well as d-,1- and meso lactide), epsilon-caprolactone, glycolide (including glycolic acid), hydroxybutyrate, hydroxyvalerate, para-dioxanone, trimethylene carbonate (and its alkyl derivatives), 1,4-dioxepan-2-one, 1,5-dioxepan-2-one, and 6,6-dimethyl-1,4-dioxan-2-one (poly(lactic acid-co-caprolactone) and poly(lactic acid-co-glycolic acid) (PLGA) are specific examples); polyether polymers and copolymers including polyarylethers such as polyphenylene ethers, polyether ketones, polyether ether ketones; polyphenylene sulfides; polyisocyanates; polyolefin polymers and copolymers, including polyalkylenes such as polypropylenes, polyethylenes (low and high density, low and high molecular weight), polybutylenes (such as polybut-1-ene and polyisobutylene), polyolefin elastomers (e.g., santoprene), ethylene propylene diene monomer (EPDM) rubbers, poly-4-methyl-pen-1-enes, ethylene-alpha-olefin copolymers, ethylene-methyl methacrylate copolymers and ethylene-vinyl acetate copolymers; fluorinated polymers and copolymers, including polytetrafluoroethylenes (PTFE), poly(tetrafluoroethylene-co-hexafluoropropene) (FEP), modified ethylene-tetrafluoroethylene copolymers (ETFE), and polyvinylidene fluorides (PVDF); silicone polymers and copolymers; polyurethanes; p-xylylene polymers; polyiminocarbonates; copoly(ether-esters) such as polyethylene oxide-polylactic acid copolymers; polyphosphazines; polyalkylene oxalates; polyoxaamides and polyoxaesters (including those containing amines and/or amido groups); polyorthoesters; biopolymers, such as polypeptides, proteins, polysaccharides and fatty acids (and esters thereof), including fibrin, fibrinogen, collagen, elastin, chitosan, gelatin, starch, and glycosaminoglycans such as hyaluronic acid; as well as blends and further copolymers of the above.

As noted above, the devices of the invention comprise a therapeutic-agent-eluting polymeric layer disposed over a first substrate surface. The therapeutic-agent-eluting polymeric layer typically contains (a) one or more polymers and (b) one or more therapeutic agents.

The therapeutic-agent-eluting polymeric layer will typically comprise, for example, from 1 wt% or less to 2 wt% to 5 wt% to 10 wt% to 25 wt% to 50 wt% or more of a single therapeutic agent or of a mixture of therapeutic agents within the layer. Therapeutic agents may be selected, for example, from those listed below, among others.

The therapeutic-agent-eluting polymeric layer will also typically comprise, for example, from 50 wt% or less to 75 wt% to 90 wt% to 95 wt% to 97.5 wt% to 99 wt% or more of a single polymer or a mixture polymers within the layer. Polymers may be biodegradable or biostable and may be selected, for example, from those described above for use in substrates and from those described below in conjunction with textured polymeric layers, among others.

The thickness of the therapeutic-agent-eluting polymeric layer may vary widely, typically ranging from 10 nm to 25 nm to 50 nm to 100 nm to 250 nm to 500nm to 1 µm to 2.5 µm to 5 µm to 10 µm to 20 µm or more in thickness.

Therapeutic-agent-eluting polymeric layers may be disposed over substrates using any suitable method known in the art. For example, where the layer contains one or more polymers having thermoplastic characteristics, the layer may be formed, for instance, by (a) providing a melt that contains polymer(s), therapeutic agent(s), and any other optional species desired and (b) subsequently cooling the melt. As another example, a layer may be formed, for instance, by (a) providing a solution or dispersion that contains one or more solvent species, polymer(s), therapeutic agent(s), and any other optional species desired and (b) subsequently removing the solvent species. The melt, solution or dispersion may be disposed on a substrate surface, for example, by extrusion onto the substrate, by co-extrusion along with the substrate, by roll-coating the substrate, by application to the substrate using a suitable application device such as a brush, roller, stamp or ink jet printer, by dipping the substrate, spray coating the substrate using spray techniques such as ultrasonic spray coating and electrohydrodynamic coating, among other methods. In some embodiments, another surface of the substrate is masked to prevent the therapeutic-agent-eluting polymeric layer from being applied thereon.

Adhesion to the substrate may be improved in some embodiments, for example, by surface modification of the substrate (e.g., a metallic substrate may be silanized to render it more hydrophobic or plasma treated to make it more hydrophilic), through the use of primer layers, or by other methods known in the art.

As noted above, the devices of the invention comprise a textured polymeric layer disposed over the substrate surface. The textured polymeric layer is textured in a way to enhance cellular attachment and/or proliferation.

The textured polymeric layer will typically comprise, for example, from 50 wt% or less to 75 wt% to 90 wt% to 95 wt% to 97.5 wt% to 99 wt% or more of a single polymer or a mixture polymers within the layer. Polymers may be biodegradable or biostable and may be selected, for example, from those listed above for use in substrates, among others. The polymer(s) may be the same as or different from the polymer(s) making up the therapeutic-acent-cluting polymeric layer.

The textured polymeric layer thickness may vary widely, typically ranging from 10 nm or less to 25 nm to 50 nm to 100 nm to 250 nm to 500nm to 1 µm to 2.5 µm to 5 µm to 10 µm to 20 µm or more in thickness.

In some embodiments, the textured polymeric layer will optionally comprise one or more therapeutic agents, comprising, for example, from 1 wt% or less to 2 wt% to 5 wt% to 10 wt% to 25 wt% to 50 wt% or more of a single therapeutic agent or of a mixture of therapeutic agents within the layer. Therapeutic agents may be selected, for example, from those listed below, with agents that promote the growth of a healthy endothelium being preferred in some embodiments.

Cell attachment, cell growth, or both, may be promoted by providing the surface with topographical features. In this regards, topographical features having a wide variety of shapes and sizes may be created within the textured polymeric layers of the invention using methods such as those discussed below. Examples of features which may be formed include the following, among others: (a) depressions whose length and width are of similar scale (e.g., pores) and whose perimeter may be of irregular or regular geometry (e.g., circular, oval, triangular, square, rectangular, pentagonal, etc.), (b) depressions whose length significantly exceeds its width (e.g., trenches and valleys), which may be, for example, of constant or variable width, and may extend along the surface in a linear fashion or in a nonlinear fashion (e.g., serpentine, zig-zag, etc.), (c) protrusions whose length and width are of similar scale (e.g., pillars, knobs, domes, mesas, protruding fibers, etc.) and whose cross-section may be regular or irregular (e.g., circular, oval, triangular, square, rectangular, pentagonal, etc.), (d) protrusions whose lengths significantly exceed their widths (e.g., ridges), which may be of constant or variable width, which may extend along the surface in a linear or nonlinear fashion; and (e) combinations of the forgoing.

Such features generally have widths that are less than 1 mm, preferably less than100 microns (µm), ranging, for example, from 100 microns or more to 75 microns to 50 microns to 40 microns to 30 microns to 20 microns to 10 microns to 5 microns or less. In some embodiments, feature widths may be between 20 and 30 microns for enhanced endothelial cell proliferation.

Features such as the above are generally spaced apart from one another by a distance of less than 1 mm, preferably less than 100 microns, for example, ranging from 100 microns or more to 75 microns to 50 microns to 40 microns to 30 microns to 20 microns to 10 microns to 5 microns or less. In some embodiments, feature spacing may be between 0.1 and 30 microns.

For example, where the texture of the polymeric layer is properly optimized (e.g., where the textural features are properly sized and spaced), endothelial cells are provided with a surface whereby proliferation to coverage (e.g., confluence) can occur rapidly.

For instance, literature has shown that endothelial cells cultured on textured surfaces spread faster and appear more like cells in native arteries. See R.G. Flemming et al., Biomaterials 20 (1999) 573-588. It has been reported that textured surfaces promote stabilized pseudo-neointima formation. In this regard, N. Fujisawa et al., Biomaterials 20 (1999) 955-962 found that, upon implantation in ovine carotid arteries, textured polyurethane surfaces consisting of regularly spaced, protruding micro-fibers on a smooth base plane (length, pitch and diameter at the base of the fibers were 250, 100 and 25 µm, respectively) promoted the formation of a stabilized thrombus base onto which subsequent cellular migration and tissue heating occurred more rapidly than onto a smooth surface.

Others have noted that by creating well-defined micro-textured patterns on a surface, fluid flow at the surface is altered to create discrete regions of low shear stress, which may serve as sanctuaries for cells such as endothelial cells and promote their retention. See S.C. Daxini et al. "Micropatterned polymer surfaces improve retention of endothelial cells exposed to flow-induced shear stress," Biorheology 2006;43(1):45-55.

Literature has shown that pore sizes in the range of 20 to 30 µm yield good endothelialization for vascular grafts.

During manufacture, polymeric layers may be applied on the substrate, for example, as preformed polymers, which preformed polymers may be bound to substrates by covalent or non-covalent forces. Where the polymer is held to the substrate by non-covalent forces, adhesion may be improved, for example, using surface modification techniques such as those discussed above. Polymeric layers may also be, for example, grown from the surface by covalently or non-covalently attaching at least one polymer precursor (e.g., a polymerization initiator) to a substrate and subsequently conducting a polymerization reaction at the surface in the presence of one or more monomers. Various techniques for producing polymer coatings through surface polymerization are described in Pub. No. US 2003/0195610 to Herrmann et al.

Texturing may be created using a number of techniques. For instance, lithographic techniques, such as photolithograpy, UV lithography, and deep-UV lithography, electron beam lithography, X-ray lithography, and nanoimprint lithography, may be used for this purpose.

For example, a patterned layer (e.g., a positive resist layer, negative resist layer, parylene based material, a microstencil, etc.) may be applied to a substrate surface, followed by application of a polymer or polymer precursor (e.g., a polymerization initiator) to the substrate in those areas not covered by the patterned layer, after which the patterned layer is removed (e.g., peeling, stripping, lift-off, etc.). In the case where a polymer precursor is applied, polymerization may be conducted before or after removal of the patterned layer.

As another example, a layer of polymer or polymer precursor material may be applied to a substrate, followed by the formation of a patterned layer (e.g., by lithography), after which the polymer or polymer precursor is removed from those areas not covered by the patterned layer (e.g., using plasma removal, solvent-based dissolution, etc.). The patterned layer is then removed. Polymerization may be conducted from the polymeric precursor material, for example, before the patterned layer is applied or after the patterned layer is removed.

As another example, a layer of polymer or polymer precursor material may be applied to a medical device substrate, followed by the direct removal and/or inactivation of certain portions of the layer (e.g., using techniques such as micromachining, nanoshaving, laser ablation, electron beam ablation, etc.). In the case where a polymer precursor is applied, polymerization may be conducted before or after the removal and/or inactivation step.

As yet another example, a layer of polymer or polymer precursor material may be directly applied to a substrate in a desired pattern (e.g., using techniques such as ink-jet printing, micro-contact printing using a suitable elastomeric stamp, microfluidic patterning and liquid-phase printing, microwriting and dip-pen nanolithography, among others. In the case where a polymer precursor is applied, this application step is followed by a suitable polymerization process.

Further information concerning various patterning techniques may be found, for example, in W. Senaratne et al., Biomacromolecules 2005, 6, 2427-244R and D. Falconnet et al., Biomaterials 27 (2006) 3044-3063.

In addition to surface topography, cell adhesion is also influenced by the chemical properties of the polymers from which the textured polymeric layers of the invention are formed.

For example, S. Sarkar et al. "Addressing thrombogenicity in vascular graft construction," J Biomed Mater Res B Appl Biomater, 2006 Oct 31; [Epub ahead of print], note that platelets adhere to positively charged surfaces due to their own negative charge as well as to hydrophobic surfaces. They suggest using hydrophilic materials with negative surface charge for synthetic graft development.

Others, such as N. Fujisawa et al., *supra,* have demonstrated that a stabilized thrombus base promotes subsequent cellular migration and tissue healing.

Surface charge is known to affect cell attachment. In this regard, see, e.g., A. Sosnik et al., "Methylation of Poloxamine for Enhanced Cell Adhesion," Biomacromolecules 2006, 7, 331-338, wherein a well-spread endothelial cell morphology was observed for positively charged methylated poloxamine films after pre-incubation in serum-containing medium. Other studies have shown that positively charged substrates are conducive to endothelial cell adhesion and morphological maturation (flattening). In this regard, see, e.g., G.L. Bowlin et al., Cell Transplant., Nov-Dec 1997, 6(6): 631-7; P.B. van Wachem et al., "Adhesion of cultured human endothelial cells onto methacrylate polymers with varying surface wettability and charge," Biomaterials, Sept. 1987, 8(5): 323-8; H. Wang et al., "Modulating cell adhesion and spreading by control of FnIII7-10 orientation on charged self-assembled monolayers (SAMs) of alkanethiolates, "J Biomed Mater Res A, 15 June 2006; 77(4): 672-8; L. Liu et al., "Controlling osteopontin orientation on surfaces to modulate endothelial cell adhesion. "J Biomed Mater Res A, 2005 Jul 1; 74(1): 23-31; and the references cited therein

Polypeptides containing cell-adhesive sequences are known to promote endothelial cell adhesion. For example, polypeptides containing RGD sequences (e.g., GRGDS) and WQPPRARI sequences are known to direct spreading and migrational properties of endothelial cells. See V. Gauvreau et al., Bioconjug Chem., 2005 Sep-Oct, 16(5), 1088-97. REDV tetrapeptide has been shown to support endothelial cell adhesion but not that of smooth muscle cells, fibroblasts, or platelets, and YIGSR pentapeptide has been shown to promote epithelial cell attachment, but not platelet adhesion. More information on REDV and YIGSR peptides can be found in U.S. Patent No. 6,156,572 and Pub. No. US 2003/0087111. A further example of a cell-adhesive sequence is NGR tripeptide, which binds to CD13 of endothelial cells. See, e.g., L. Holle et al., "In vitro targeted killing of human endothelial cells by co-incubation of human serum and NGR peptide conjugated human albumin protein bearing alpha (1-3) galactose epitopes," Oncol. Rep. 2004 Mar; 11(3):613-6.

Other polymer blocks useful for cell adhesion may be selected from suitable proteins, glycoproteins, polysaccharides, proteoglycans, glycosaminoglycans and subunits of the same, for example, those set forth in Pub. No. US 2005/0187146 to Helmus et al.

While certain polymer blocks, including several of those described above, are known to result in specific and non-specific adsorption, among other effects, other polymer blocks (e.g., polyethylene glycol blocks) are known to prevent non-specific adsorption and may be provided within the textured polymeric layers for this purpose in some embodiments of the invention.

Polymer blocks affecting cell adhesion and/or proliferation, such as those above, among others, may be, for example, adsorbed to the textured polymeric layer, covalently attached to the textured polymeric layer, disposed within textured polymeric layer (e.g., provided along with one or more additional polymers in the textured polymeric layer, provided as one or more polymer blocks within a block copolymer in the textured polymeric layer, etc.).

Recently, sub-micron topographic features have been shown to influence the behavior of a variety of cell types. See, e.g., the review by E.K.F Yim et al., "Significance of synthetic nanostructures in dictating cellular response," Nanomedicine: Nanotechnology, Biology, and Medicine 1 (2005) 10- 21 and Viitala R. et al., "Surface properties of in vitro bioactive and non-bioactive sol-gel derived materials," Biomaterials, 2002 Aug; 23(15): 3073-86.

Submicron topography, including pores, fibers, and elevations in the sub-100 nm range, has also been observed for the basement membrane of the aortic valve endothelium as well as for other basement membrane materials. See R.G. Flemming et al., Biomaterials 20 (1999) 573-588, S. Brody et al., Tissue Eng. 2006 Feb; 12(2): 413-421, and S.L. Goodman et al., Biomaterials 1996; 17: 2087-95.

In some embodiments of the invention, textured polymeric layers are employed which inherently provide sub-micron surface features as a result of phase separation. For example, the textured polymeric layer may comprise two or more polymer blocks that phase separate from one another. The blocks may, for example, correspond to separate polymers or they may be present within the same polymer (e.g., as blocks within a block copolymer).

Polymer blocks include low and high glass transition temperature (Tg) polymer blocks. In general, low Tg polymer blocks are soft and elastomeric at room (and body) temperature, whereas high Tg polymer blocks are hard. As used herein, "low Tg polymer blocks" are those that display a Tg that is below body temperature, more typically 37°C to 20°C to 0°C to -25°C to -50°C or below. Conversely, elevated or "high Tg polymer blocks" are those that display a glass transition temperature that is above body temperature, more typically 37°C to 50°C to 75°C to 100°C or above. Tg can be measured by any of a number of techniques including differential scanning calorimetry (DSC). Examples of low Tg blocks include low Tg polyalkylene blocks, low Tg polysiloxane blocks, low Tg poly(halogenated alkylene) blocks, low Tg polyacrylate blocks, low Tg polymethacrylate blocks, low Tg poly(vinyl ether) blocks, and low Tg poly(cyclic ether) blocks, among others. Examples of high Tg blocks include vinyl aromatic blocks, such as those made from styrenic monomers, high Tg polyacrylate blocks, high Tg polymethacrylate blocks, poly(vinyl alcohol) blocks, high Tg poly(vinyl ester) blocks, high Tg poly(vinyl amine) blocks, high Tg poly(vinyl halide) blocks, high Tg poly(alkyl vinyl ethers), and high Tg polyamide blocks, among others.

Polymer blocks include hydrophobic and hydrophilic polymer blocks. Examples of hydrophobic polymer blocks include those that are glassy or partially crystalline at the application temperature, for instance, high Tg hydrophobic blocks such as polystyrene and its derivatives, polyacrylates having alkyl side chains, and so forth. Examples of hydrophobic polymer blocks also include those that are soft at the application temperature, for instance, low Tg hydrophobic blocks such as polyalkylene blocks, poly(halogenated alkylene) blocks, polysiloxane blocks, and so forth. Examples of hydrophilic polymer blocks also include materials such as polypeptides or their synthetic derivatives, polyalkylene oxides (e.g., PEO), ionic polymers including polyelectrolytes, and so forth. Further hydrophobic and hydrophilic blocks may be selected from those described elsewhere herein.

In some embodiments, the textured polymeric layer comprises polymer brushes. As used herein, a "polymer brush" is made up of polymers that are directly or indirectly tethered to a surface, which polymers contain one or more ends that are free to extend from the surface, somewhat analogous to the bristles of a brush. In the devices of the present invention, the polymer brushes comprise two or more polymer blocks of differing composition, which are sometimes referred to as "mixed polymer brushes".

Such polymer brushes commonly undergo a process known as perpendicular segregation. For example, upon exposure to a hydrophobic environment (e.g., exposure to a relatively nonpolar organic solvent such as toluene), the surface of a polymer brush containing hydrophobic and hydrophilic blocks may become more hydrophobic due to the migration of the hydrophobic blocks to the surface (and the migration of hydrophilic blocks away from the surface), whereas upon exposure to a hydrophilic environment (e.g., exposure to an aqueous environment or exposure to a relatively polar organic solvent such as methanol or ethanol), the surface may become more hydrophilic due to the migration of the hydrophilic blocks to the surface (and the migration of hydrophobic blocks away from the surface). As a result of this ability to change properties, such polymer brushes are sometimes referred to a stimulus responsive, "switchable" or "smart".

Stimulus responsive polymer brushes commonly fall into one of two categories. In a first category, one or more types of hydrophobic blocks and one or more types of hydrophilic blocks (e.g., a mixture of hydrophilic and hydrophobic chains) are separately attached to the surface. In the second category, one or more hydrophobic blocks and one or more hydrophilic blocks are provided within a single block copolymer that is attached to the surface. Examples of the latter copolymers include the following: CBL, CLB and BCL, among others, where C is a covalent linking entity for linkage to the substrate surface, B is a hydrophobic block, and L is a hydrophilic block. Alternatively, polymers may be attached to the substrate by non-covalent interactions. Examples include the following block copolymers: PBL, PLB and BPL, among others, where P is a polymeric block which is capable of adsorption on the substrate, and B and L are defined above. Note that a combination of hydrophilic polymer blocks L and hydrophobic polymer blocks B may also be may be provided, for example, along the backbone of a polymer block P or a polymeric covalent linking entity C, among other configurations.

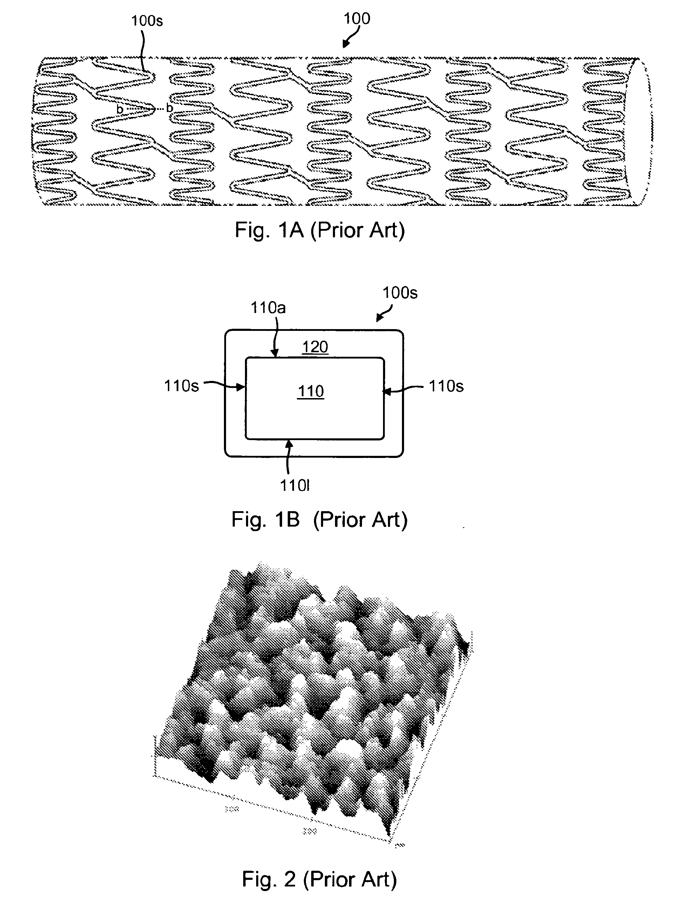

Stimulus responsive polymer brushes are known to display both lateral and perpendicular phase segregation on a submicron scale. For example, Fig. 2 is a high resolution (300 nm x 300 nm) atomic force microscopy topography image of a surface grafted Y-shaped binary polymer molecule with one hydrophobic polystyrene arm, one hydrophilic polyacrylic acid arm and a short anchoring junction. From M.C. LeMieux et al., Biomacromolecules, Vol. 17, No. 1,2006, 331-338. Such features have a scale that is similar to features associated with endothelium basement membrane materials.

Various techniques for forming polymer brushes are described, for example, in U.S. Serial No. 11/388,652, including those that are covalently attached and those that are non-covalently attached to a substrate. Covalent attachment of polymers to form polymer brushes is commonly achieved by "grafting to" and "grafting from" techniques. "Grafting to" techniques commonly involve tethering pre-formcd end-functionalized polymer blocks to a suitable substrate under appropriate conditions. "Grafting from" techniques, on the other hand, typically involve covalently immobilizing initiators on the substrate surface, followed by surface initiated polymerization to generate the polymer brushes. Each of these techniques involves the attachment of a species (e.g., a polymer or polymer precursor, typically, an initiator) to a surface, which may be carried out using a number of known techniques. These include covalent coupling of species to a substrate surface, each having reactive functional groups, which may be carried out by direct reaction between the functional groups, or through the use of linking/coupling agents that contain reactive moieties capable of reaction with such functional groups. Specific examples of known linking agents include glutaraldehyde, diisocyanates, diiosothiocyanates, bis(hydroxysuccinimide)esters, maleimidehydroxysuccinimide esters, carbodiimides, N,N'-carbonyldiimidazole imidoesters, and difluorobenzene derivatives, among others. Further information on covalent coupling may be found, for example, in Pub. No. US 2005/0002865. For many substrates, including polymeric substrates, surface functional groups may be introduced by treating the substrate with a reactive plasma. Surface functional groups may also be introduced by conducting plasma polymerization processes in which so-called "monomers" are employed, which contain functional groups. By using a second feed gas (commonly a non-polymerizable gas) in combination with the unsaturated monomer, it is possible to incorporate this second species in the plasma deposited layer as well. Examples of gases that may be used include, allylamine, pyridine, nitroethane, ethylene oxide, allylalcohol, ethylene glycol monomethyl ether, acrylic acid, n-vinyl pyrrolidone, acetylene/H₂O, acetylene/CO, acetylene/N₂, acetylene/CO/H₂O, acetylene/N₂/ H₂O, ethylene/N₂, allylamine/NH₃, acetylene/SO₂, ethylenC/SO₂, and acrylic acid/CO₂, among others. Further information concerning plasma functionalization may be found, for example, in "Functionalization of Polymer Surfaces," Europlasma Technical Paper, 05/08/04 and in Pub. No. US 2003/0236323. For conductive substrates, electrochemical processes may be employed for attachment of polymers or initiators. In this regard, technology for linking an initiator to an electrically conductive surface, including metallic substrate materials such as those discussed above (e.g., stainless steel, nitinol, etc.), is disclosed by Claes et al., "Polymer Coating of Steel by a Combination of Electrografting and Atom-Transfer Radical Polymerization," Macromolecules, Web release No. 0217130, published July 19, 2003 and in Pub. No. US 2006/0013853, filed 7/19/2004 and entitled "Medical Devices Having Conductive Substrate and Covalently Bonded Coating Layer." In general, the initiator will have at least one functionality that is conducive to electrografting and at least one functionality that is able to initiate free radical polymerization (*e.g*., an activated halide functionality, which is able to initiate ATRP polymerization of, for example, unsaturated monomers). One specific example of such a species is 2-chloropropionate ethyl acrylate (cPEA).

As noted above, in the "grafting from" process once an initiator is attached to the surface, a polymerization reaction is then conducted to create a surface bound polymer. Various polymerization reactions may be employed, including various condensation, anionic, cationic and radical polymerization methods. These and other methods may be used to polymerize a host of monomers and monomer combinations. Specific examples of radical polymerization processes are controlled/living" radical polymerizations such as metal-catalyzed atom transfer radical polymerization (ATRP), stable free-radical polymerization (SFRP), nitroxide-mediated processes (NMP), and degenerative transfer (e.g., reversible addition-fragmentation chain transfer (RAFT)) processes, among others. The advantages of using a "living" free radical system for polymer brush creation include control over the brush thickness via control of molecular weight and narrow polydispersities, and the ability to prepare block copolymers by the sequential activation of a dormant chain end in the presence of different monomers. These methods are well-detailed in the literature and are described, for example, in an article by Pyun and Matyjaszewski, "Synthesis ofNanocomposite Organic/Inorganic Hybrid Materials Using Controlled/"Living" Radical Polymerization," Chem. Mater., 13:3436-3448 (2001).

A few specific examples of techniques which have been used to produce brush polymers are described below. One example of a "grafting to" techniques is described in D. Usov et al., "Mixed Polymer Brushes with Thermal Response Amplified by Roughness," Polymeric Materials: Science & Engineering 2004, 90, 622-623, in which the following polymers were covalently attached to amino groups on PTFE substrates, via their end carboxylic groups,: α,ω-dicarboxy-terminated poly(styrene-co-2,3,4,5,(-pentafluorostyrene) (PSF), α,ω-dicarboxy-terminated poly(methylacrylate-co-1,1,1,3,3,3-hexafluoroisopropyl methacrylate) (PHFA), and carboxy terminated poly(N-isopropyl acrylamide) (PNiPAAm). Switching ability of the synthesized mixed polymer brushes upon exposure to selective solvents was observed (toluene is selective for PSF and/or PHFA, whereas ethanol is selective for PNiPAAm), as was thermally induced switching. An example of a "grafting from" technique is described in M. Motornov et al., "Mixed Polymer Brushes on Polyamide Substrates," Polymeric Materials: Science & Engineering 2004, 88, 264-265. In this technique, polyamide (PA) samples were first treated with NH₃ plasma. An azo-initiator, 4,4'-azobis(4-cianopentanoic acid), was then covalently grafted to the plasma modified substrate, via the reaction of the amino-groups on the substrate with the hydroxy-groups on the initiator. Graft polymerization of polystyrene chains and poly(2-vinylpyridine) chains was then conducted. A pronounced switching effect upon exposure to toluene and ethanol was observed. Igor Luzinov et al., National Textile Center Annual Report: November 2003 describe forming a mixed polymer brush of polystyrene and polyacrylic acid chains and on a poly(glycidylmethacrylate) (PGMA) substrate using a combination of the "grafting to" and "grafting from" techniques. The brushes changed their surface morphology, when they were exposed to solvents with different polarity. Luzinov et al. also describe a technique whereby Y-shaped block copolymers, which contain polystyrene and polyacrylic acid arms and an aromatic functional stem having a reactive carboxylate group, are grafted to the substrate surface. It was observed that these arms are capable of local reversible rearrangements leading to a reversible surface structural reorganization in different solvents. Analogously, switchable diblock and triblock polymers may be grafted to substrates using "grafting to" methods, "grafting from" methods, and combinations of the same. For an example of a diblock copolymer exhibiting switchable behavior see, e.g., S A Prokhorova, et al., "Can polymer brushes induce motion of nano-objects'?" 2003 Nanotechnology 14 1098-1108, in which poly(methyl methacrylate-*b-*glycidyl methacrylate) diblock-copolymer brushes are synthesized by "grafting from" a covalently attached initiator on the surface of a silicon wafer. See also Pub. No. US 2003/0219535 in which nitroxide mediated free radical polymerization of vaporized vinyl monomers, including acrylic acid (AAc), styrene (St), N-2-(hydroxypropyl) methacrylamide (HPMA) and N-isopropyl acrylamide (NIPAAm), on silicon wafers is demonstrated. For example, a poly(AAc)- poly(St)-poly(HPMA) triblock copolymer is synthesized.

Submicron surface features may thus be produced using block copolymers that contain two or more chains of differing composition. Block copolymeric configurations may vary widely and include, for example, the following configurations, among others, which comprise one or more hydrophilic polymer chains designated "L" and one or more hydrophobic polymer chains designated "B": (a) block copolymers having alternating chains of the type (BL)ₘ, B(LB)ₘ and L(BL)ₘ, where m is a positive whole number of 1 or more, (b) block copolymers such as X(BL)ₘ or X(LB)ₘ where X is a hub species (e.g., an initiator molecule residue, a linking residue, etc.), (c) comb copolymers having an B chain backbone and multiple L side chains and (d) comb copolymers having an L chain backbone and multiple B side chains.

Submicron surface features may also be produced using bottle-brush copolymers having a main chain and a mixture of hydrophilic and hydrophobic side chains, tapered copolymers (e.g., polymers in which the monomer composition gradually changes from hydrophobic to hydrophilic along the chain length and in which the chain ends are essentially homopolymeric), and hyperbranched copolymers having hydrophobic and hydrophilic portions.

Submicron surface features may be further produced using by bombarding the surface with ions, by vapor deposition techniques, by techniques in which a precursor region comprising a material present in nano-domains is subjected to conditions under which the material is either reduced in volume or eliminated from the precursor region, by physical vapor deposition processes, by ion deposition, by X-ray lithography, by kinetic metallization, by electrodeposition, or by electroless deposition, among various other processes. See, e.g., Pub. Nos. US 2006/0129215 to Helmus et al., US 2006/0127443 to Helmus et al. and US 2006/0171985 to Richard et al.

In other embodiments, submicron surface features may be produced using a textured mold. One specific embodiment, which employs a nanotextured mold, follows. A bare metal stent may be coated with a suitable biostable or biodisintegrable polymer such as SIBS or PLGA. A compacted hollow or solid cylinder (or mandrel) of nanoparticles (e.g., titania or another suitable material) is also formed by compacting the nanoparticles in a mold and sintering at a low temperature to create a compact with a nanotextured surface. The diameter of the compacted cylinder may be made to be slightly less than that of the coated stent. The compacted cylinder is placed inside the coated stent, after which the stent is compressed radially onto the cylinder. A vacuum may be created around the assembly, if desired. The temperature is then raised to just below the softening (or glassy) temperature of the polymer. At this point, the temperature is raised just above the softening temperature for a brief period to enhance imprinting from the nanotextured mold. The temperature is then reduced and the stent removed. The foregoing procedure imprints a nanotexture onto the polymer coating on the inside of the stent. Alternatively a metal hollow or solid cylinder can be anodized or etched to create a mandrel with a nano-rough surface. See, e.g., C. Yao et al., "Increased Osteoblast Adhesion on Nano-rough Anodized Titanium and CoCrMo", Technical Proceedings of the 2006 Nanotechnology Conference and Trade Show, Vol. 2, pp. 119-122. Different nano-features can be created using different metals, for example anodizing Ti can create a pattern of nanotubes of TiO₂ on the surface of the mandrel. See, e.g., A. Kar et al., Surface & Coatings Technology, 201 (2006) 3723-3731.

In order to release the mandrel after the imprinting process has completed, the mandrel can be shrunk by reducing the temperature. Alternatively the mandrel can be made in the shape of a chuck (e.g., a collet) that is expanded during imprinting and reduced for removal.

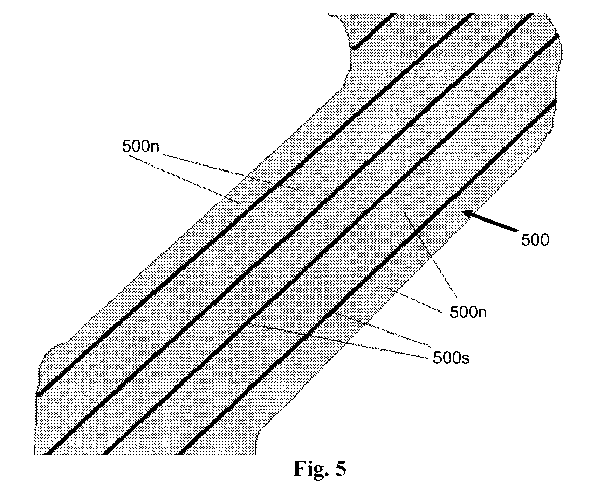

In a blood vessel, endothelial cells (which are elongated) align themselves with the blood flow. See, e.g., V. Fuster et al., Hurst's The Heart, 11th Ed., 2004, Chapter 7, Biology of the Vessel Wall, pp. 135 *et seq.* In various vascular embodiments of the invention, including the formation of vascular stents, it is desired encourage this alignment on the device surface. Such features may also encourage motility and adhesion of the endothelial cells, encouraging stent encapsulation, particularly in long or overlapping stents where endothelial cell colonization may progress from the ends inwards. Adjacent nanotextured and non-nanotextured areas may thus facilitate motility, adhesion, and/or alignment of the endothelial cells. Such features may be created by creating smooth areas on the nanotextured mandrel (such as one of those described above) using laser polishing or other localized heating mechanism, and the resulting mandrel can be used as a mold as described above. These features can preferably be narrow linear strips (e.g., alternating smooth and nanotextured strips ranging from 1 to 50 microns (µm) in width) created in the same direction as the longitudinal axis of the stent. For example, referring now to Fig. 5, a luminal surface of a portion of a stent strut 500 is schematically shown. The surface comprises nanotextured regions 500n separated by non-nanotextured (e.g., smooth) regions 500s. The regions 500n, 500s extend along the luminal surface of the stent in a direction that is parallel to the longitudinal axis of the stent in the form of strips. Such features may also be created directly on a nanotextured polymeric stent surface by heating selective portions of the polymeric surface to melt the same.

In other embodiments, polymeric surfaces may be etched to impart submicron surface features. Etching has been reported to alter the chemical and/or topographical characteristics of polymers, including the creation of submicron surface features. For instance, polymers based on acidic monomers have been etched with basic solutions and polymers based on basic monomers have been etched with acidic solutions to create nanoscale surface features. See J. B. Thomas et al., Chapter 22 "Nanotechnology and Biomaterials," Nanomaterials Handbook, Y. Gogotsi, Ed., CRC Press and the references cited therein. For example, chemical etching of biodegradable polymers such as polylactide (PLA), polyglycolide (PGA), poly(lactide-co-glycolide) (PLGA) and polycaprolactone have been reported in basic solution, particularly NaOH. Chemical etching of poly(ether)urethanes, on the other hand, have been reported in acidic solution, particularly HNO₃. See, e.g., D.C. Miller et al., Biomaterials 25 (2004) 53-61.

Regardless of how the submicron features are formed, the resulting features (e.g., depressions, protrusions, etc.) may have widths that are less than 1 micron, ranging, for example, from 1000 nm to 500 nm to 250 nm to 100 nm to 75 nm to 50 nm to 40 nm to 30 nm to 20 nm to 10 to 5 nm or less in width.

Another parameter that is known to influence cell adhesion is peptide clustering at the nanoscale level. See M. Tirrell et al., "The role of surface science in bioengineered materials," Surface Science 500 (2002) 61-83. Phase separation (e.g., into submicron hydrophobic phases and submicron hydrophilic phases that contain polypeptide cell-adhesive sequences) will likewise result in peptide clustering on a nanoscale.

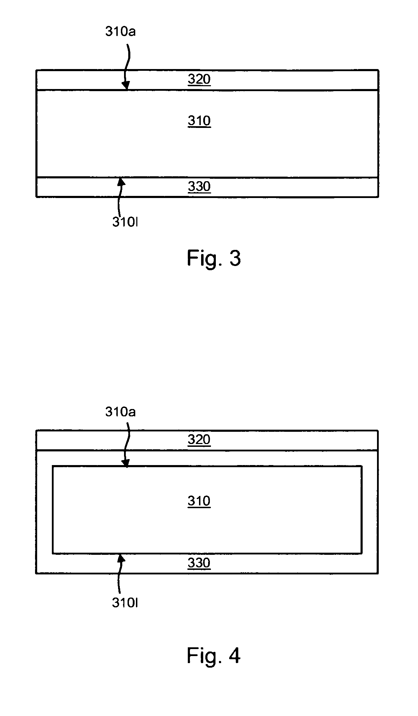

Two exemplary embodiments of the invention will now be described in conjunction with Figs. 3 and 4.

Fig. 3 is a cross-sectional view of a stent strut (e.g., analogous to the cross-sectional view taken along line b--b of strut 110s of stent 100 of Fig. 1A), in accordance with an embodiment of the invention. As opposed to the cross-section of Fig. 1A, however, in the cross-section of Fig. 3, the stent comprises a therapeutic-agent-eluting polymeric layer 320 disposed over a first surface of the stent substrate 310 (i.e., the abluminal surface 310a) and a textured polymeric layer 330 (e.g., one comprising topographical features whose widths are less than 100 µm) disposed over a second surface of the stent substrate 310 (i.e., the luminal surface 3101). Such an embodiment may be formed, for example, by first forming a textured polymeric layer 330 over the luminal surface 3101 of the stent substrate 310, followed by formation of a therapeutic-agent-eluting polymeric layer 320 over the abluminal surface 310a, or vice versa. This may be done with or without masking using techniques such as those described above.

Like Fig. 3, Fig. 4 is a cross-section of a stent strut in accordance with an embodiment of the invention. Moreover, the stent comprises a therapeutic-agent-eluting polymeric layer 320 disposed over the abluminal surface 310a of the stent substrate 310 and a textured polymeric layer 330 disposed over the luminal surface 3101 of the stent substrate 310. However, in Fig. 4, the textured polymeric layer 330 is further disposed on the lateral surfaces of the stent substrate 310, and it is disposed on the abluminal surface 110a between the stent substrate 310 and the therapeutic-agent-eluting polymeric layer 320 as well. Such an embodiment may be formed, for example, by first forming a textured polymeric layer 330 over the entire surface of the stent, followed by the formation of a therapeutic-agent-eluting polymeric layer 320 over only the abluminal surface 310a of the stent substrate 310 (e.g., with or without masking). Such an embodiment is advantageous, for example, in that it may provide an adhesion layer for the polymer layer 320.

As noted above, a wide variety of therapeutic agents may be employed in conjunction with the present invention, including genetic therapeutic agents, non-genetic therapeutic agents and cells, which may be used for the treatment of a wide variety of diseases and conditions. Numerous therapeutic agents are described here.

Suitable non-genetic therapeutic agents for use in connection with the present invention may be selected, for example, from one or more of the following: (a) antithrombotic agents such as heparin, heparin derivatives, urokinase, clopidogrel, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anti-coagulants such as D-Phe-Pro-Arg chloromethyl ketone, an RGD peptide-containing compound, heparin, hirudin, antithrombin compounds, platelet receptor antagonists, anti-thrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (1) antimicrobial agents such as triclosan, cephalosporins, antimicrobial peptides such as magainins, aminoglycosides and nitrofurantoin; (m) cytotoxic agents, cytostatic agents and cell proliferation affectors; (n) vasodilating agents; (o)agents that interfere with endogenous vasoactive mechanisms, (p) inhibitors of leukocyte recruitment, such as monoclonal antibodies; (q) cytokines; (r) hormones; (s) inhibitors of HSP 90 protein (i.e., Heat Shock Protein, which is a molecular chaperone or housekeeping protein and is needed for the stability and function of other client proteins/signal transduction proteins responsible for growth and survival of cells) including geldanamycin, (t) beta-blockers, (u) bARKct inhibitors, (v) phospholamban inhibitors, (w) Serca 2 gene/protein, (x) immune response modifiers including aminoquizolines, for instance, imidazoquinolines such as resiquimod and imiquimod, (y) human apolioproteins (e.g., AI, AII, AIII, AIV, AV, etc.).

Preferred non-genetic therapeutic agents include paclitaxel (including particulate forms thereof, for instance, protein-bound paclitaxel particles such as albumin-bound paclitaxel nanoparticles, e.g., ABRAXANE), sirolimus, everolimus, tacrolimus, Epo D, dexamethasone, estradiol, halofuginone, cilostazole, geldanamycin, ABT-578 (Abbott Laboratories), trapidil, liprostin, Actinomcin D, Resten-NG, Ap-17, abciximab, clopidogrel, Ridogrel, beta-blockers, bARKct inhibitors, phospholamban inhibitors, Serca 2 gene/protein, imiquimod, human apolioproteins (e.g., AI-AV), growth factors (e.g., VEGF-2), as well a derivatives of the forgoing, among others.

Exemplary genetic therapeutic agents for use in connection with the present invention include anti-sense DNA and RNA as well as DNA coding for: (a) anti-sense RNA, (b) tRNA or rRNA to replace defective or deficient endogenous molecules, (c) angiogenic factors including growth factors such as acidic and basic fibroblast growth factors, vascular endothelial growth factor, epidermal growth factor, transforming growth factor α and β, platelet-derived endothelial growth factor, platelet-derived growth factor, tumor necrosis factor α, hepatocyte growth factor and insulin-like growth factor, (d) cell cycle inhibitors including CD inhibitors, and (e) thymidine kinase ("TK") and other agents useful for interfering with cell proliferation. Also of interest is DNA encoding for the family of bone morphogenic proteins ("BMP's"), including BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Vectors for delivery of genetic therapeutic agents include viral vectors such as adenoviruses, gutted adenoviruses, adeno-associated virus, retroviruses, alpha virus (Semliki Forest, Sindbis, etc.), lentiviruses, herpes simplex virus, replication competent viruses (e.g., ONYX-015) and hybrid vectors; and non-viral vectors such as artificial chromosomes and mini-chromosomes, plasmid DNA vectors (e.g., pCOR), cationic polymers (e.g., polyethyleneimine, polyethyleneimine (PEI)), graft copolymers (e.g., polyether-PEI and polyethylene oxide-PEI), neutral polymers PVP, SP1017 (SUPRATEK), lipids such as cationic lipids, liposomes, lipoplexes, nanoparticles, or microparticles, with and without targeting sequences such as the protein transduction domain (PTD).

Cells for use in connection with the present invention include cells of human origin (autologous or allogeneic), including whole bone marrow, bone marrow derived mono-nuclear cells, progenitor cells (e.g., endothelial progenitor cells), stem cells (e.g., mesenchymal, hematopoietic, neuronal), pluripotent stem cells, fibroblasts, myoblasts, satellite cells, pericytes, cardiomyocytes, skeletal myocytes or macrophage, or from an animal, bacterial or fungal source (xenogeneic), which can be genetically engineered, if desired, to deliver proteins of interest.

Numerous therapeutic agents, not necessarily exclusive of those listed above, have been identified as candidates for vascular and other treatment regimens, for example, as agents targeting restenosis. Such agents are useful for the practice of the present invention and suitable examples may be selected from one or more of the following: (a) Ca-channel blockers including benzothiazapines such as diltiazem and clentiazem, dihydropyridines such as nifedipine, amlodipine and nicardapine, and phenylalkylamines such as verapamil, (b) serotonin pathway modulators including: 5-HT antagonists such as ketanserin and naftidrofuryl, as well as 5-HT uptake inhibitors such as fluoxetine, (c) cyclic nucleotide pathway agents including phosphodiesterase inhibitors such as cilostazole and dipyridamole, adenylate/Guanylate cyclase stimulants such as forskolin, as well as adenosine analogs, (d) catecholamine modulators including α-antagonists such as prazosin and bunazosine, β-antagonists such as propranolol and α/β-antagonists such as labetalol and carvedilol, (e) endothelin receptor antagonists, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomers), as well as C-nitroso-compounds, O-nitroso-compounds, N-nitroso-compounds and L-arginine, (g) Angiotensin Converting Enzyme (ACE) inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including cilostazole, aspirin and thienopyridine (ticlopidine, clopidogrel) and GP IIb/IIIa inhibitors such as abciximab, epitifibatide and tirofiban, (k) coagulation pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (1) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM-I and ICAM-I interactions, (r) prostaglandins and analogs thereof including prostaglandins such as PGE1 and PGI2 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, fluvastatin, simvastatin and cerivastatin, (u) fish oils and omega-3 -fatty acids, (v) free-radical scavengers/antioxidants such as probucol, vitamins C and E, ebselen, trans-retinoic acid and SOD mimics, (w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-[beta] pathway agents such as polyanionic agents (heparin, fucoidin), decorin, and TGF-[beta] antibodies, EGF pathway agents such as EGF antibodies, receptor antagonists and chimeric fusion proteins, TNF-[alpha] pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives, (x) MMP pathway inhibitors such as marimastat, ilomastat and metastat, (y) cell motility inhibitors such as cytochalasin B, (z) antiproliferative/antineoplastic agents including antimetabolites such as purine analogs (e.g., 6-mercaptopurine or cladribine, which is a chlorinated purine nucleoside analog), pyrimidine analogs (e.g., cytarabine and 5-fluorouracil) and methotrexate , nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, Epo D, paclitaxel and epothilone), caspase activators, proteasome inhibitors, angiogenesis inhibitors (e.g., endostatin, angiostatin and squalamine), rapamycin, cerivastatin, flavopiridol and suramin, (aa) matrix deposition/organization pathway inhibitors such as halofuginone or other quinazolinone derivatives and tranilast, (bb) endothelialization facilitators such as VEGF and RGD peptide, and (cc) blood rheology modulators such as pentoxifylline. [0094] Numerous additional therapeutic agents useful for the practice of the present invention are also disclosed in U.S. Patent No. 5,733,925 to Kunz et al.

## Claims

1. A medical device comprising: (a) a substrate having first and second surfaces, (b) a therapeutic-agent-eluting polymeric layer disposed over the first substrate surface that comprises a first therapeutic agent, and (c) a textured polymeric layer disposed over the second surface that is textured to promote cell attachment, cell growth, or both, wherein the medical device is an implantable or insertable device, wherein the medical device is exposed to blood upon implantation or insertion, wherein the medical device is a stent having a longitudinal axis, **characterised in that** the textured polymeric layer has a plurality of elongated alternating textured and non-textured surface regions in a direction that is parallel to the longitudinal axis of the stent.

2. The medical device of claim 1, wherein the first surface is the abluminal surface of the stent, and wherein the second surface is the luminal surface of the stent.

3. The medical device of claim 1 wherein the first therapeutic agent is an antiproliferative agent that discourages cell growth or wherein the first therapeutic agent is selected atom paclitaxel and other taxanes, rapamycin and rapamycin analogs.

4. The medical device of claim 1, wherein the textured polymeric layer comprises a second therapeutic agent that differs from the first therapeutic agent.

5. The medical device of claim 3, wherein the textured polymeric layer comprises an endothelial cell growth promoter, in particular wherein the endothelial cell growth promoter is a vascular endothelial growth factor (VEGF).

6. The medical device of claim 1, wherein the therapeutic-agent-eluting polymeric layer is biostable or wherein the therapeutic-agent-eluting polymeric layer is biodisintegrable, or wherein the therapeutic-agent-eluting polymeric layer comprises a block copolymer.

7. The medical device of claim 6, wherein the block copolymer is selected from block copolymers comprising vinyl aromatic blocks and olefin blocks, block copolymers comprising acrylate blocks and methacrylate blocks, and block copolymers comprising olefin blocks and methacrylate blocks.

8. The medical device of claim 7, wherein the block copolymer is selected from a poly(styrene-[epsilon]-isobutylene-[epsilon]-styrene) triblock copolymer, a poly (methyl methacrylate-[epsilon]- butyl acrylate-[epsilon]-methyl methacrylate) triblock copolymer, and a poly (methyl methacrylate-[epsilon]-isobutylene-[epsilon]-methyl methacrylate) triblock copolymer.

9. The medical device of claim 1, wherein the textured polymeric layer comprises topographical features that are separated from one another by distances within the range of 0.01 to 100 µm or wherein the textured polymeric layer comprises topographical features having widths and spacings ranging between 1 nm and 100 nm.

10. The medical device of claim 1, wherein the textured polymeric layer is biostable.

11. The medical device of claim 1, wherein the textured polymeric layer comprises a copolymer selected from block copolymers, dendritic copolymers, tapered copolymers, and bottlebrush copolymers.

12. The medical device of claim 1, wherein the textured and non-textured surface regions are aligned in the direction of blood flow that occurs upon implantation or insertion of the medical device.

13. The medical device of claim 1, wherein the textured polymeric layer comprises polymer blocks and hydrophobic polymer blocks that segregate to form said topographical features.

14. The medical device of claim 6, wherein the textured polymeric layer comprises hydrophilic polymer blocks and hydrophobic polymer blocks.

15. The medical device of claim 13, wherein the copolymer is covalently attached to the substrate.

## Patentansprüche

1. Medizinische Vorrichtung umfassend: (a) ein Substrat mit ersten und zweiten Oberflächen, (b) eine ein therapeutisches Mittel freisetzende Polymerschicht, angeordnet über der ersten Substratoberfläche, welche ein erstes therapeutisches Mittel umfasst, und (c) eine über der zweiten Oberfläche angeordnete strukturierte Polymerschicht, welche dazu strukturiert ist, Zellanhaftung, Zellwachstum oder beides zu begünstige, wobei die medizinische Vorrichtung ein implantierbares oder einsetzbares Gerät ist, wobei das medizinische Gerät bei Implantation oder Einsetzung Blut ausgesetzt wird und wobei das medizinische Gerät ein Stent mit einer Längsachse ist, **dadurch gekennzeichnet dass** die strukturierte Polymerschicht eine Vielzahl von in einer zu der Längsachse des Stents parallelen Richtung langgstreckten, alternierenden strukturierten und nicht strukturierten Oberflächengebieten aufweist.

2. Medizinische Vorrichtung gemäß Anspruch 1, wobei die erste Oberfläche die abluminale Oberfläche des Stents ist und wobei die zweite Oberfläche die luminale Oberfläche des Stents ist.

3. Medizinische Vorrichtung gemäß Anspruch 1, wobei das erste therapeutische Mittel ein Gewebevermehrung hemmendes Mittel ist, welches Zellwachstum entgegenwirkt, oder wobei das erste therapeutische Mittel aus Paclitaxel und anderen Taxanen, Rapamycin und Rapamycinanalogen ausgewählt ist.

4. Medizinische Vorrichtung gemäß Anspruch 1, wobei die strukturierte Polymerschicht ein zweites therapeutisches Mittel umfasst, welches sich von dem ersten therapeutischen Mittel unterscheidet.

5. Medizinische Vorrichtung gemäß Anspruch 3, wobei die strukturierte Polymerschicht einen Beschleuniger für das Wachstum von Endothelzellen umfasst, insbesondere wobei der Beschleuniger für das Wachstum von Endothelzellen ein vaskulärer endothelialer Wachstumsfaktor (VEGF) ist.

6. Medizinische Vorrichtung gemäß Anspruch 1, wobei die ein therapeutisches Mittel freisetzende Polymerschicht biostabil ist oder wobei die ein therapeutisches Mittel freisetzende Polymerschicht biologisch zersetzbar ist oder wobei die ein therapeutisches Mittel freisetzende Polymerschicht ein Blockcopolymer umfasst.

7. Medizinische Vorrichtung gemäß Anspruch 6, wobei das Blockcopolymer aus Blockcopolymeren umfassend vinylaromatische Blöcke und Olefinblöcke, Blockcopolymeren umfassend Acrylatblöcke und Methacrylatblöcke und Blockcopolymeren umfassend Olefinblöcke und Methacrylatblöcke ausgewählt ist.

8. Medizinische Vorrichtung gemäß Anspruch 7, wobei das Blockcopolymer aus einem Styrol-[epsilon]-Isobutylen-[epsilon]-Styrol)-Triblockcopolymer, einem Methylmethacrylat-[epsilon]-Butylacrylat-[epsilon]-Methylmethacrylat)-Triblockcopolymer und einem Methylmethacrylat-[epsilon]-Isobutylen-[epsilon]-Methylmethacrylat)-Triblockcopolymer ausgewählt ist.

9. Medizinische Vorrichtung gemäß Anspruch 1, wobei die strukturierte Polymerschicht topographische Merkmale umfasst, welche durch Abstände innerhalb des Bereichs von 0,01 bis 100 µm voneinander getrennt sind, oder wobei die strukturierte Polymerschicht topographische Merkmale mit Breiten und Abständen umfasst, die im Bereich zwischen 1 nm und 100 nm liegen.

10. Medizinische Vorrichtung gemäß Anspruch 1, wobei die strukturierte Polymerschicht biostabil ist.

11. Medizinische Vorrichtung gemäß Anspruch 1, wobei die strukturierte Polymerschicht ein Polymer ausgewählt aus Blockcopolymeren, dendritischen Copolymeren, Gradienten-Copolymeren und Kammcopolymeren umfasst.

12. Medizinische Vorrichtung gemäß Anspruch 1, wobei die strukturierten und nicht strukturierten Oberflächengebiete in der Richtung der Blutströmung ausgerichtet sind, welche bei Implantation oder Einsetzung der medizinischen Vorrichtung stattfindet.

13. Medizinische Vorrichtung gemäß Anspruch 1, wobei die strukturierte Polymerschicht Polymerblöcke und hydrophobe Polymerblöcke umfasst, welche sich entmischen, um die topographischen Merkmale auszubilden.

14. Medizinische Vorrichtung gemäß Anspruch 6, wobei die strukturierte Polymerschicht hydrophile Polymerblöcke und hydrophobe Polymerblöcke umfasst.

15. Medizinische Vorrichtung gemäß Anspruch 13, wobei das Copolymer kovalent an das Substrat gebunden ist.

## Revendications

1. Dispositif médical comportant : (a) un substrat avec premières et deuxièmes surfaces, (b) une surface polymérique éluant un agent thérapeutique, disposée au-dessus de la première surface de substrat et comportant un premier agent thérapeutique, et (c) une couche polymérique texturée disposée au-dessus de la deuxième surface, qui est texturée à favoriser l'attachement de cellules, la croissance de cellules ou les deux, le dispositif médical étant un dispositif implantable ou insérable, le dispositif médical étant exposé au sang pendant l'implantation ou insertion, et le dispositif médical étant un Stent avec un axe longitudinal, **caractérisé en ce que** la couche polymérique texturée a une pluralité de régions de surface texturées et non texturées, allongées dans une direction qui est parallèle à l'axe longitudinal du Stent.

2. Dispositif médical selon la revendication 1, dans lequel la première surface est la surface abluminale du Stent et la deuxième surface est la surface luminale du Stent.

3. Dispositif médical selon la revendication 1, dans lequel le premier agent thérapeutique est un agent antiproliferatif qui freine l'accroissement de tissu, ou dans lequel le premier agent thérapeutique est sélectionné de paclitaxel et autres taxanes, rapamycine et analogues de rapamycine.

4. Dispositif médical selon la revendication 1, dans lequel la couche polymérique texturée comporte un deuxième agent thérapeutique qui est différent du premier agent thérapeutique.

5. Dispositif médical selon la revendication 3, dans lequel la couche polymérique texturée comporte un promoteur de croissance de cellules endothéliques, en particulier quand le promoteur de la croissance de cellules endothéliques est un facteur de croissance de l'endothélium vasculaire (VEGF).

6. Dispositif médical selon la revendication 1, dans lequel la couche polymérique éluant un agent thérapeutique est bio-stable ou dans lequel la couche polymérique éluant un agent thérapeutique est bio-désintégrable ou dans laquelle la couche polymérique éluant un agent thérapeutique comporte un copolymère en bloc.

7. Dispositif médical selon la revendication 6, dans lequel le copolymère en bloc est sélectionné de copolymères en bloc comportant des blocs vinylaromatiques et des blocs oléfiniques, copolymères en bloc comportant des blocs d'acrylate et des blocs de méthacrylate, et de copolymères en bloc comportant des blocs oléfiniques et des blocs de méthacrylate.

8. Dispositif médical selon la revendication 7, dans lequel le copolymère en bloc est sélectionné d'un copolymère tribloc Styrène-[epsilon]-Isobutylène-[epsilon]-Styrène, un copolymère tribloc Méthylméthacrylate-[epsilon]-Butylacrylate-[epsilon]-Méthylméthacrylate et un copolymère tribloc Méthylméthacrylate-[epsilon]-Isobutylène-[epsilon]- Méthylméthacrylate.

9. Dispositif médical selon la revendication 1, dans lequel la couche polymérique texturée comporte des caractéristiques topographiques qui sont séparées l'une de l'autre par des distance dans la gamme de 0,01 à 100 µm, ou dans lequel la couche polymérique texturée comporte des caractéristiques topographiques avec largeurs et écarts dans la gamme entre 1 nm et 100 nm.

10. Dispositif médical selon la revendication 1, dans lequel la couche polymérique texturée est bio-stable.

11. Dispositif médical selon la revendication 1, dans lequel la couche polymérique texturée comporte un polymère sélectionné de copolymères en bloc, copolymères dendritiques, copolymères cunéiformes et copolymères en peigne.

12. Dispositif médical selon la revendication 1, dans lequel les régions de surface texturées et non texturées sont alignées dans la direction du courant de sang qui a lieu dans l'implantation ou insertion du dispositif médical.

13. Dispositif médical selon la revendication 1, dans lequel la couche polymérique texturée comporte des blocs de polymère et des blocs de polymère hydrophobes qui se séparent pour former les caractéristiques topographiques.

14. Dispositif médical selon la revendication 6, dans lequel la couche polymérique texturée comporte des blocs de polymère hydrophiles et des blocs de polymère hydrophobes.

15. Dispositif médical selon la revendication 13, dans lequel le copolymère est attaché au substrat de façon covalente.
